# EUROPEAN PATENT APPLICATION

(11) **EP 4 674 358 A1**
(43) Date of publication of application: **07.01.2026**
(21) Application number: 24779873.9
(22) Date of filing: 21.03.2024
(51) Int. Cl.: A61B 8/12, A61B 1/00

(54) **TOMOGRAPHIC IMAGE GENERATION DEVICE AND TOMOGRAPHIC IMAGE GENERATION SYSTEM**

(30) Priority: 28.03.2023 JP 2023052327
(71) Applicant: Terumo Kabushiki Kaisha, Tokyo 151-0072 (JP)
(72) Inventor: UEHARA, Ryo, Ashigarakami-gun, Kanagawa 259-0151 (JP)
(74) Representative: Casalonga
(86) International application number: PCT/JP2024/011113
(87) International publication number: WO 2024/203754

(57) **Abstract**

A tomographic image generation apparatus drives a sensor unit of a probe and generates a tomographic image of a luminal organ, and includes an ultrasound signal transceiver that transmits an ultrasound signal to the sensor unit and receives a reflected signal reflected by the luminal organ and output from the sensor unit, a signal processing circuit that generates ultrasound tomographic image data of the luminal organ based on the reflected signal received by the ultrasound signal transceiver, and a wireless communication circuit that transmits ultrasound tomographic image data generated by the signal processing circuit to a display apparatus.

## Description

### Technical Field

The present invention relates to a tomographic image generation apparatus and a tomographic image generation system.

### Background Art

As minimally invasive treatment for ischemic heart diseases such as angina pectoris or myocardial infarction, intravascular treatment represented by percutaneous coronary intervention (PCI) is performed. Intravascular image diagnosis apparatuses such as an ultrasound image diagnosis apparatus (intraVascular ultra sound (IVUS)) using ultrasound and an optical coherence image diagnosis apparatus (optical coherence tomography (OCT)) using near infrared rays are used for preoperative diagnosis or confirmation of results after surgery in endovascular treatment. An image diagnosis apparatus includes an image diagnosis catheter, a drive device that rotates a sensor unit provided in the image diagnosis catheter in a circumferential direction, and a console. The console includes a control device, an operation unit, a monitor screen, and the like (for example, Patent Literature 1).

Note that an image diagnosis apparatus combining a function of the IVUS and a function of the OCT, that is, an image diagnosis apparatus including an ultrasound transmitter and receiver capable of transmitting/receiving ultrasound and an optical transmitter and receiver capable of transmitting/receiving light has been proposed.

### Citation List

### Patent Literature

Patent Literature 1: WO 2014/162368 A

### Summary of Invention

### Technical Problem

A console of an image diagnosis apparatus is a device having a large size corresponding to a human, and has a large occupied area with respect to a catheter chamber, which has a problem of hindering procedure.

An object of the present disclosure is to provide a tomographic image generation apparatus and a tomographic image generation system capable of generating ultrasound tomographic image data on a drive device side and wirelessly transmitting the ultrasound tomographic image data to an external display device without using a console.

### Solution to Problem

A tomographic image generation apparatus according to one aspect of the present disclosure is (1) a tomographic image generation apparatus that drives a sensor unit of a probe and generates a tomographic image of a luminal organ, the tomographic image generation apparatus including: an ultrasound signal transceiver that transmits an ultrasound signal to the sensor unit and receives a reflected signal reflected by the luminal organ and output from the sensor unit; a signal processing circuit that generates ultrasound tomographic image data of the luminal organ based on the reflected signal received by the ultrasound signal transceiver; and a wireless communication circuit that transmits the ultrasound tomographic image data generated by the signal processing circuit to a display apparatus.

The tomographic image generation apparatus according to (1) preferably includes (2) a probe connection portion to which the probe is detachably connected; a light source device connection portion to which an optical device is detachably connected, the optical device transmitting measurement light for obtaining an optical coherence tomographic image of the luminal organ and converting, into an electric signal, interference light based on reflected light reflected by the luminal organ and output from the sensor unit; and an optical connection portion that optically connects the optical device and the probe, the signal processing circuit preferably generates the optical coherence tomographic image of the luminal organ based on the electric signal converted by the optical device, and the wireless communication circuit preferably transmits optical coherence tomographic image data generated by the signal processing circuit to the display apparatus.

The tomographic image generation apparatus according to (1) or (2) preferably includes (3) an optical path length control device that outputs, to the optical device, a control signal for controlling an optical path length of reference light for obtaining the optical coherence tomographic image of the luminal organ.

In the tomographic image generation apparatus according to any one of (1) to (3), (4) the light source device connection portion preferably includes a control signal line connection portion to which a control signal line for transmitting a control signal for controlling operation of the optical device is connected, an optical fiber connection portion to which an optical fiber for transmitting the measurement light for obtaining the optical coherence tomographic image and the reflected light is connected, and a detection signal line connection portion to which a detection signal line for transmitting the electric signal related to the interference light is connected.

In the tomographic image generation apparatus according to any one of (1) to (4), (5) in the light source device connection portion, the control signal line connection portion, the optical fiber connection portion, and the detection signal line connection portion are preferably connected to the control signal line, the optical fiber, and the detection signal line by one connector.

In the tomographic image generation apparatus according to any one of (1) to (5), preferably, (6) the light source device connection portion includes a power supply connection portion to which a power supply line through which power supplied from the optical device is transmitted is connected, and the tomographic image generation apparatus includes: a battery mounted inside; and a power supply circuit that, in a case where power supplied from an outside is received, supplies the received power to the signal processing circuit, and, in a case where the power supplied from the outside is not received, supplies power of the battery to the signal processing circuit.

The tomographic image generation apparatus according to any one of (1) to (6) preferably includes (7) a power supply circuit that supplies power of a battery mounted inside to the signal processing circuit.

The tomographic image generation apparatus according to any one of (1) to (7) preferably includes (8) a power supply circuit that receives power supplied from an outside and supplies the received power to the signal processing circuit.

The tomographic image generation apparatus according to any one of (1) to (8) preferably includes (9) a rotation drive mechanism that rotates the sensor unit of the probe, and a motor that applies power to the rotation drive mechanism.

The tomographic image generation apparatus according to any one of (1) to (9) preferably includes (10) a storage unit that stores a plurality of pieces of ultrasound tomographic image data corresponding to a plurality of positions, in a longitudinal direction, of the luminal organ.

A tomographic image generation system according to one aspect of the present disclosure preferably includes: the tomographic image generation apparatus according to any one of (1) to (10); a catheter having a sensor unit that scans a luminal organ with ultrasound or light; and an optical device that transmits measurement light for obtaining an optical coherence tomographic image of the luminal organ, receives interference light based on reflected light reflected by the luminal organ and output from the sensor unit, and converts the interference light into an electric signal.

### Advantageous Effects of Invention

According to the present disclosure, it is possible to eliminate a console, generate ultrasound tomographic image data on a drive device side and wirelessly transmit the ultrasound tomographic image data to an external display device.

### Brief Description of Drawings

Fig. 1 is an explanatory diagram illustrating a configuration example of an image diagnosis system.
Fig. 2 is an explanatory diagram illustrating a configuration example of the image diagnosis system from which an image diagnosis catheter and an optical device are removed.
Fig. 3 is an explanatory diagram illustrating a configuration example of the image diagnosis catheter.
Fig. 4 is a block diagram illustrating a configuration example of the image diagnosis system.
Fig. 5 is a block diagram illustrating a configuration example of a signal processing circuit.
Fig. 6 is an explanatory diagram schematically illustrating a cross-section of a blood vessel through which a sensor unit is inserted.
Fig. 7A is an explanatory diagram of a tomographic image.
Fig. 7B is an explanatory diagram of the tomographic image.

### Description of Embodiments

Hereinafter, a tomographic image generation apparatus and a tomographic image generation system of the present disclosure will be described in detail based on the drawings illustrating embodiments thereof. The following respective embodiments will describe cardiac catheter treatment that is intravascular treatment as an example. The present invention is not limited by these examples but is disclosed in the claims, and is intended to include all changes within the meaning and scope equivalent to the claims. At least some of the embodiments described below may be combined as appropriate.

In the present embodiment, an image diagnosis system (tomographic image generation system) using a dual type catheter having functions of both intravascular ultrasound (IVUS) and optical coherence tomography (OCT) will be described. In the dual type catheter, a mode of acquiring an ultrasound tomographic image only by IVUS, a mode of acquiring an optical coherence tomographic image only by OCT, and a mode of acquiring both tomographic images by IVUS and OCT are provided, and these modes can be switched and used. Hereinafter, the ultrasound tomographic image and the optical coherence tomographic image are appropriately referred to as an IVUS image and an OCT image, respectively. In addition, the IVUS image and the OCT image will be collectively referred to as a tomographic image.

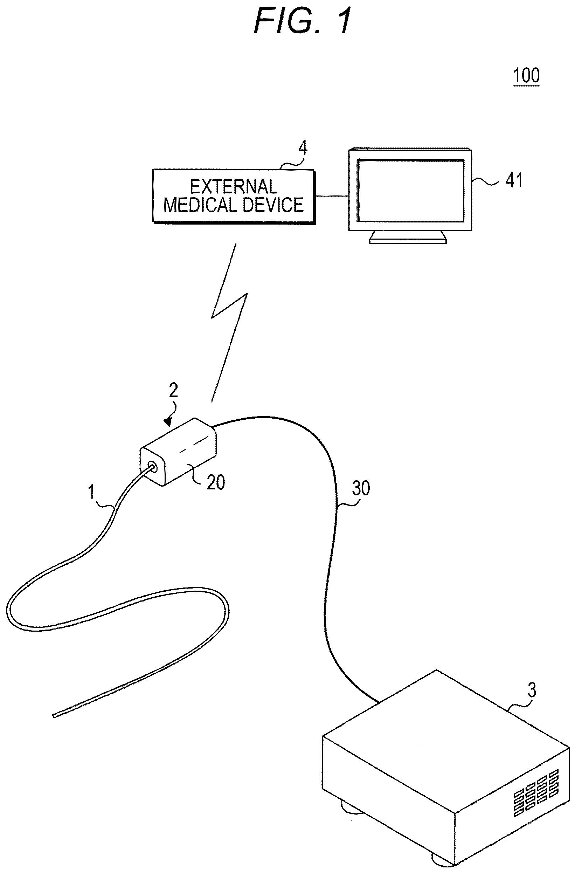

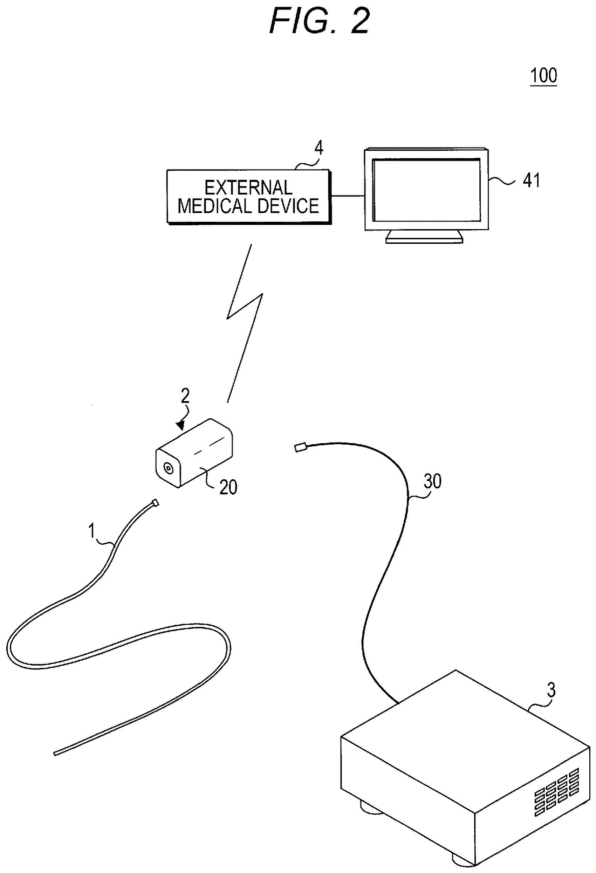

Fig. 1 is an explanatory diagram illustrating a configuration example of an image diagnosis system 100, and Fig. 2 is an explanatory diagram illustrating a configuration example of the image diagnosis system 100 from which an image diagnosis catheter 1 and a light source device 3 are removed. The image diagnosis system 100 of the present embodiment includes the image diagnosis catheter 1, an image diagnosis apparatus 2, a light source device 3, and an external medical device 4 having a display apparatus 41. The image diagnosis system 100 according to the present embodiment is a system in which a large console including an optical system and a display monitor for implementing an OCT function is eliminated and the image diagnosis apparatus 2 is downsized. It is difficult to downsize the optical system for implementing the OCT function, and thus, the light source device 3 having the optical system is separated from the image diagnosis apparatus 2, and as illustrated in Figs. 1 and 2, the light source device 3 can be attached to and detached from the image diagnosis apparatus 2 as necessary. In addition, the image diagnosis apparatus 2 is configured to perform wireless communication with the external medical device 4 and display an IVUS image and an OCT image on a display apparatus 41 of the external medical device 4. The image diagnosis apparatus 2 configured as described above is an apparatus configured by an electric circuit that mainly executes electric signal processing and data processing, and thus can be downsized.

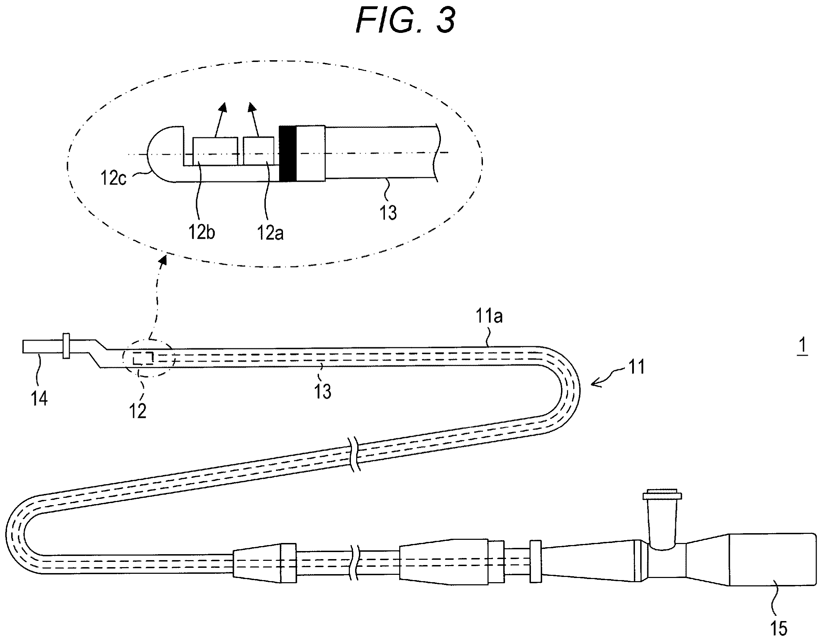

Fig. 3 is an explanatory diagram illustrating a configuration example of the image diagnosis catheter 1. Note that a region surrounded by a one-dot chain line in an upper part of Fig. 3 is an enlarged view of a region surrounded by a one-dot chain line in a lower part. The image diagnosis catheter 1 includes a probe 11 and a probe connector 15 disposed at an end portion of the probe 11. The probe 11 is connected to the image diagnosis apparatus 2 via the probe connector 15. In the following description, a side far from the probe connector 15 of the image diagnosis catheter 1 will be referred to as a distal end side, and a side of the probe connector 15 will be referred to as a proximal end side. The probe 11 includes a catheter sheath 11a, and a guide wire insertion portion 14 through which a guide wire can be inserted is provided at a distal portion thereof. The guide wire insertion portion 14 constitutes a guide wire lumen, receives a guide wire inserted in advance into a blood vessel, and guides the probe 11 to an affected part by the guide wire. The catheter sheath 11a forms a tube portion continuous across a range from the guide wire insertion portion 14 to the probe connector 15. A shaft 13 is inserted into the catheter sheath 11a, and a sensor unit 12 is connected to a distal end side of the shaft 13.

The sensor unit 12 includes a housing 12c, and a distal end side of the housing 12c is formed in a hemispherical shape in order to suppress friction and catching with an inner surface of the catheter sheath 11a. In the housing 12c, an optical transmitter and receiver 12a that transmits near-infrared light into the blood vessel and receives reflected light from an inside of the blood vessel, and an ultrasound transmitter and receiver 12b that transmits ultrasound into the blood vessel and receives reflected waves from the blood vessel are disposed. In the example illustrated in Fig. 3, the ultrasound transmitter and receiver 12b is provided on the distal end side of the probe 11, and the optical transmitter and receiver 12a is provided on the proximal end side. In other words, the optical transmitter and receiver 12a and the ultrasound transmitter and receiver 12b are arranged in the housing 12c so as to be separated by a predetermined length along an axial direction on a central axis of the shaft 13 (on a two-dot chain line in Fig. 3).

In addition, the optical transmitter and receiver 12a and the ultrasound transmitter and receiver 12b are arranged such that transmission/reception directions of near-infrared light and ultrasound are directions of approximately 90 degrees with respect to the axial direction of the shaft 13 (radial direction of the shaft 13). Note that the optical transmitter and receiver 12a and the ultrasound transmitter and receiver 12b are desirably attached slightly shifted from the radial direction so as not to receive the reflected wave and reflected light on the inner surface of the catheter sheath 11a. In the present embodiment, for example, as indicated by an arrow in Fig. 3, the optical transmitter and receiver 12a is attached with a direction inclined to the proximal end side with respect to the radial direction as an irradiation direction of the near-infrared light, and the ultrasound transmitter and receiver 12b is attached with a direction inclined to the distal end side with respect to the radial direction as an irradiation direction of the ultrasound.

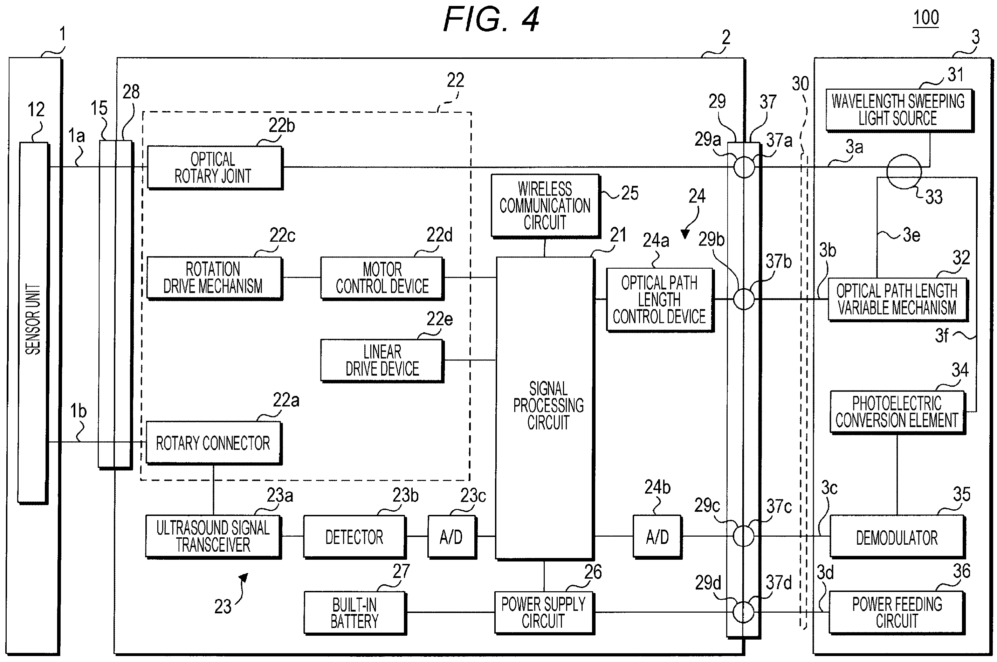

An optical fiber cable 1a (see Fig. 4) connected to the optical transmitter and receiver 12a and an electric signal cable 1b (see Fig. 4) connected to the ultrasound transmitter and receiver 12b are inserted into the shaft 13. The probe 11 is inserted into the blood vessel from the distal end side. The sensor unit 12 and the shaft 13 can move forward or rearward inside the catheter sheath 11a and can rotate in a circumferential direction. The sensor unit 12 and the shaft 13 rotate about the central axis of the shaft 13 as a rotation axis.

Fig. 4 is a block diagram illustrating a configuration example of the image diagnosis system 100. The light source device 3 is a device including an optical system for obtaining an OCT image using coherence of laser light. Here, a wavelength sweeping type optical system will be described, but a scheme of an optical measurement method is not particularly limited. A spectral domain type or other optical systems may be provided. The wavelength sweeping type light source device 3 according to the present embodiment includes a wavelength sweeping light source 31, an optical path length variable mechanism 32, an optical coupler 33, a photoelectric conversion element 34, a demodulator 35, a power feeding circuit 36, and a light source connector 37. The light source device 3 houses these optical systems in an optical system housing. It is preferable that the optical system housing can be disposed under a bed of a patient who is to receive catheter treatment.

The light source connector 37 includes an optical terminal 37a, a control signal terminal 37b, a detection signal terminal 37c, and a power supply terminal 37d. The light source connector 37 includes the respective terminals in one connector housing. In other words, a user (medical worker) can connect the optical fiber 3a, the control signal line 3b, the detection signal line 3c, and the power supply line 3d to be described later to the image diagnosis apparatus 2 only by connecting one light source connector 37 to the image diagnosis apparatus 2. The optical fiber 3a, the control signal line 3b, the detection signal line 3c, and the power supply line 3d are bundled to constitute one connection cable 30.

The wavelength sweeping light source 31 is a light source that generates laser light having a wavelength that is continuously changed. The wavelength sweeping light source 31 includes, for example, a light source, a diffraction grating, and a polygon mirror. The light from the light source is dispersed by the diffraction grating and enters a surface of the polygon mirror, and only light having a wavelength orthogonal to the polygon mirror returns through the same optical path. Time sweeping of the wavelength can be performed by rotating the polygon mirror. The light subjected to wavelength-time sweeping is output through an optical coupler for light source (not illustrated).

One end of the optical fiber 3a is connected to the wavelength sweeping light source 31. An optical coupler 33 is provided in the middle of the optical fiber 3a, and the other end of the optical fiber 3a is connected to an optical terminal 37a of the light source connector 37. The optical fiber 3a is coupled to an optical fiber 3e, an optical fiber 3f, and the optical system by the optical coupler 33. The light output from the wavelength sweeping light source 31 is branched by the optical coupler 33, and the branched first light (hereinafter, referred to as measurement light) is externally output from the optical terminal 37a via the optical fiber 3a. As will be described later, the measurement light externally output is output to the image diagnosis apparatus 2, and the blood vessel is irradiated with the measurement light through the image diagnosis apparatus 2 and the probe 11. The reflected light reflected in the blood vessel is input to the light source device 3 via the image diagnosis apparatus 2 and the optical fiber 3a.

One end of the optical fiber 3e is connected to the optical path length variable mechanism 32, and the other end of the optical fiber 3f is connected to the photoelectric conversion element 34. Second light (hereinafter, referred to as reference light) branched by the optical coupler 33 is transmitted through the optical fiber 3e. The optical path length variable mechanism 32 is a mechanism that finely adjusts an optical path length of the reference light. The optical path length variable mechanism 32 includes optical path length changing means for changing an optical path length corresponding to variation in length so as to be able to absorb variation in length of each of the image diagnosis catheter 1 and the probe 11 when the image diagnosis catheter 1 and the probe 11 are replaced. The optical path length variable mechanism 32 includes a collimator lens, a reflecting mirror, a uniaxial stage, and the like. The uniaxial stage changes the optical path length by changing a position of the collimator lens or the reflecting mirror. The reference light incident on the optical path length variable mechanism 32 propagates through the optical path having a length that is adjusted by the optical path length variable mechanism 32. The reference light reflected by the reflecting mirror is emitted from the optical path length variable mechanism 32 and transmitted through the optical fiber 3f. The reference light and the reflected light are multiplexed by the optical coupler 33, and the multiplexed interference light enters the photoelectric conversion element 34 via the optical fiber 3f.

One end of the control signal line 3b is connected to the optical path length variable mechanism 32, and the other end of the control signal line 3b is connected to the control signal terminal 37b of the light source connector 37. The optical path length variable mechanism 32 operates in accordance with a control signal input via the control signal terminal 37b and the control signal line 3b. The control signal is a signal output from the image diagnosis apparatus 2 as described later.

The photoelectric conversion element 34 photoelectrically converts the interference light and outputs the photoelectrically converted signal to the demodulator 35. The demodulator 35 demodulates the signal of the interference light. One end of the detection signal line 3c is connected to an output end of the demodulator 35, and the other end of the detection signal line 3c is connected to the detection signal terminal 37c of the light source connector 37. The demodulator 35 externally outputs a signal (hereinafter, referred to as a detection signal) obtained by demodulating the signal of the interference light from the detection signal terminal 37c. As will be described later, the externally output detection signal is input to the image diagnosis apparatus 2.

The power feeding circuit 36 is a circuit that supplies power for driving the image diagnosis apparatus 2. One end of the power supply line 3d is connected to the power feeding circuit 36, and the other end of the power supply line 3d is connected to the power supply terminal 37d of the light source connector 37. The power feeding circuit 36 externally outputs power via the power supply line 3d and the power supply terminal 37d. As will be described later, the power output from the light source device 3 is supplied to the image diagnosis apparatus 2.

The image diagnosis apparatus 2 includes a housing 20 (see Fig. 1 and 2), a signal processing circuit 21, a motor drive unit (MDU) 22, an IVUS-related circuit 23, an OCT-related circuit 24, a wireless communication circuit 25, a power supply circuit 26, a built-in battery 27, a probe connection portion 28, and a light source device connection portion 29. The housing 20 houses the signal processing circuit 21, the MDU 22, the IVUS-related circuit 23, the OCT-related circuit 24, the wireless communication circuit 25, the power supply circuit 26, the built-in battery 27, the probe connection portion 28, and the light source device connection portion 29 described above.

The probe connection portion 28 is a connector to which the probe connector 15 of the image diagnosis catheter 1 is detachably connected.

The light source device connection portion 29 is a connector to which the light source connector 37 of the light source device 3 is detachably attached. The light source device connection portion 29 includes an optical fiber connection portion 29a to which the optical fiber 3a is connected, a control signal line connection portion 29b to which the control signal line 3b is connected, a detection signal line connection portion 29c to which the detection signal line 3c is connected, and a power supply connection portion 29d to which the power supply line 3d is connected.

The MDU 22 is a drive device that drives a built-in motor according to operation by the user and controls the operation of the image diagnosis catheter 1 inserted into the blood vessel. The MDU 22 includes a rotary connector 22a, an optical rotary joint (optical connection portion) 22b, a rotation drive mechanism 22c, a motor control device 22d, and a linear drive device 22e.

The rotary connector 22a is an electrical connector that rotatably connects the electric signal cable 1b connected to the ultrasound transmitter and receiver 12b and an ultrasound signal transceiver 23a. The rotary connector 22a includes a rotary electrode portion having a sliding contact and a fixed electrode portion, the fixed electrode is connected to the ultrasound signal transceiver 23a, and the electric signal cable 1b is connected to the rotary electrode.

The optical rotary joint 22b is an optical connector that connects the optical fiber cable 1a connected to the optical transmitter and receiver 12a and the optical fiber connection portion 29a of the light source device connection portion 29. The optical rotary joint 22b has a rotating portion and a fixed portion, the fixed portion is connected to the optical fiber connection portion 29a by an internal optical fiber cable, and the optical fiber cable 1a is connected to the rotating portion.

The rotation drive mechanism 22c includes a motor that rotates the rotary electrode of the rotary connector 22a and the fixed portion of the optical rotary joint 22b. By driving the motor of the rotation drive mechanism 22c, the sensor unit 12 and the shaft 13 inserted into the probe 11 can be rotated in the circumferential direction. The rotation of the motor is controlled by the motor control device 22d. Further, the rotation drive mechanism 22c includes an encoder that detects a rotation angle of the motor, and the encoder outputs a rotation angle signal indicating the rotation angle of the motor to the motor control device 22d.

The motor control device 22d outputs a synchronization control signal to the rotation drive mechanism 22c and the signal processing circuit 21. The rotation drive mechanism 22c rotates the motor in accordance with the synchronization control signal output from the motor control device 22d. In addition, the motor control device 22d outputs the rotation angle signal output from the rotation drive mechanism 22c to the signal processing circuit 21.

The linear drive device 22e includes a motor that moves the sensor unit 12 and the shaft 13 inserted into the probe 11 in the axial direction. The operation of the linear drive device 22e is controlled by the signal processing circuit 21.

According to the MDU 22 configured in this manner, it is possible to perform pull-back operation of rotating the sensor unit 12 and the shaft 13 inserted into the probe 11 in the circumferential direction while pulling the sensor unit 12 and the shaft 13 toward the MDU 22 side at a constant speed. The sensor unit 12 can continuously scan the inside of the blood vessel at predetermined time intervals while moving and rotating from the distal end side to the proximal end side by the pull-back operation, and the signal processing circuit 21 can continuously generate a plurality of tomographic images substantially perpendicular to the probe 11 based on the scanning results.

Note that while the MDU 22 having a pull-back function has been described, the MDU 22 may be configured not to have the pull-back function. In other words, the MDU 22 may be configured not to include the linear drive device 22e. Note that in a case where the pull-back mechanism is eliminated, the user manually pulls the MDU 22.

The IVUS-related circuit 23 includes an ultrasound signal transceiver 23a, a detector 23b, and an A/D converter 23c. The ultrasound signal transceiver 23a transmits an ultrasound signal for generating an IVUS image. The ultrasound signal is, for example, a pulse wave. The ultrasound signal transceiver 23a transmits an ultrasound signal to the ultrasound transmitter and receiver 12b via the rotary connector 22a. In addition, the ultrasound signal transceiver 23a receives a reflected wave emitted into the blood vessel and reflected. The reflected wave received by the ultrasound signal transceiver 23a is detected by the detector 23b. The A/D converter 23c converts the detected analog reflected signal into digital data.

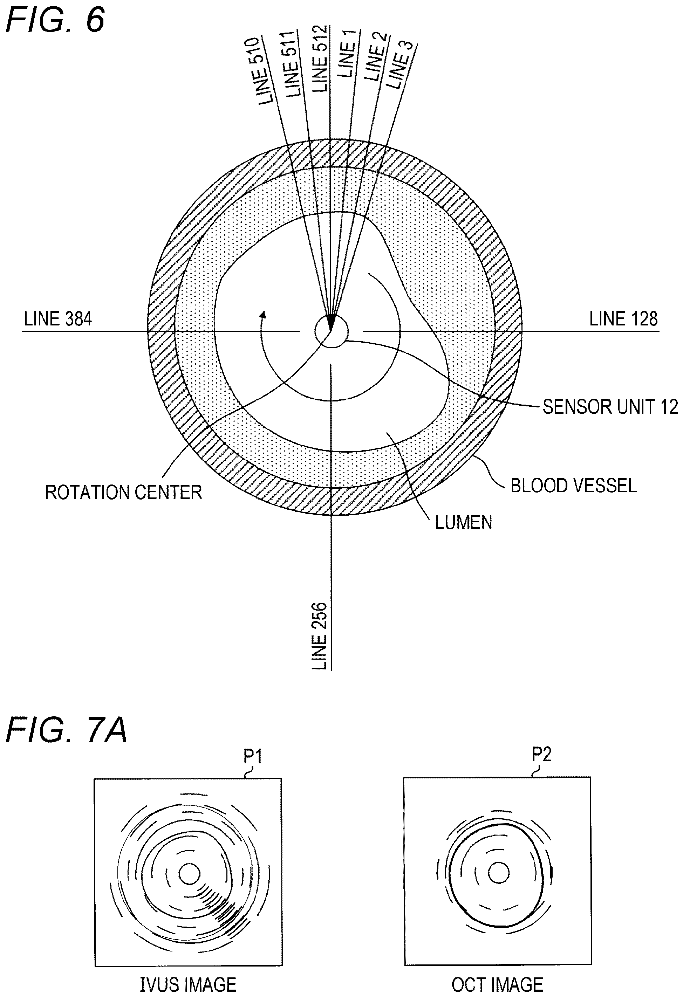

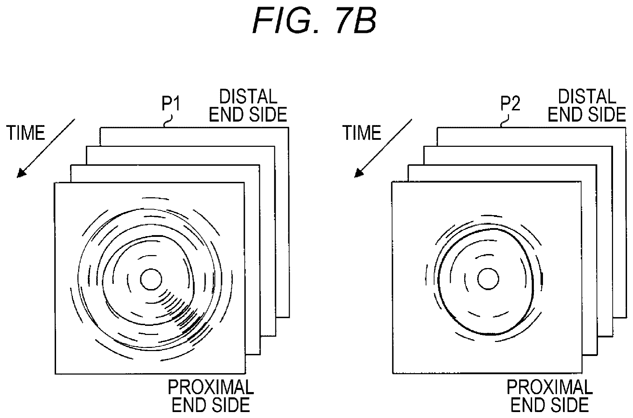

The signal processing circuit 21 samples the reflected wave signal at a predetermined rate to generate digital ultrasound line data (ultrasound tomographic image data) from the reflected signal. The ultrasound line data is data indicating reflection intensity of the ultrasound in a depth direction of the blood vessel viewed from the ultrasound transmitter and receiver 12b. An IVUS image P1 (see Figs. 7A and 7B) representing a transverse section of the blood vessel can be constructed based on the generated ultrasound line data.

The OCT-related circuit 24 includes an optical path length control device 24a and an A/D converter 24b. The optical path length control device 24a is a circuit that outputs a control signal for controlling operation of the optical path length variable function of the light source device 3. The optical path length control device 24a is connected to the control signal line connection portion 29b. The optical path length control device 24a outputs a control signal to the optical path length variable mechanism 32 via the control signal line connection portion 29b to adjust an optical path length of the reference light.

The A/D converter 24b is connected to a detection signal line connection portion 29c. The A/D converter 24b converts an analog detection signal input via the detection signal line 3c and the detection signal line connection portion 29c into digital data.

The signal processing circuit 21 samples the detection signal at a predetermined rate to generate digital optical line data (optical coherence tomographic image data) from the detection signal. The image diagnosis apparatus 2 can construct an OCT image P2 (see Figs. 7A and 7B) representing a transverse section of the blood vessel based on the generated optical line data.

The wireless communication circuit 25 includes a communication circuit that performs wireless communication with the external medical device 4. The wireless communication standards and protocol are not particularly limited. In addition, the wireless communication circuit 25 may be configured to perform wireless communication directly with the external medical device 4 or may be configured to perform communication via a router. The wireless communication circuit 25 can wirelessly transmit the ultrasound line data and the optical line data provided from the signal processing circuit 21 to the external medical device 4.

Note that the wireless communication circuit 25 may wirelessly transmit the ultrasound line data and the optical line data to the external medical device 4, or may wirelessly transmit frame data of an IVUS image P1 and an OCT image P2 configured as two-dimensional frame images to the external medical device 4 as the ultrasound tomographic image data and the optical coherence tomographic image data.

The power supply circuit 26 is a circuit that supplies power to the signal processing circuit 21 and other circuits constituting the image diagnosis apparatus 2. The image diagnosis apparatus 2 includes a built-in battery 27, and the power supply circuit 26 supplies power of the built-in battery 27 to the signal processing circuit 21, and the like. The built-in battery 27 may be a primary battery or a secondary battery. The power supply circuit 26 may include a charging circuit for the built-in battery 27 that is a secondary battery.

The power supply circuit 26 is connected to the power supply connection portion 29d and can receive power supplied from the light source device 3 via the power supply line 3d. The power supply circuit 26 supplies power supplied from the light source device 3 to the signal processing circuit 21, and the like.

In a case where the light source device 3 is connected and power is externally supplied, the power supply circuit 26 supplies the externally supplied power to the signal processing circuit 21, and the like, instead of the power of the built-in battery 27. In a case where the light source device 3 is not connected, the power of the built-in battery 27 is supplied to the signal processing circuit 21, and the like.

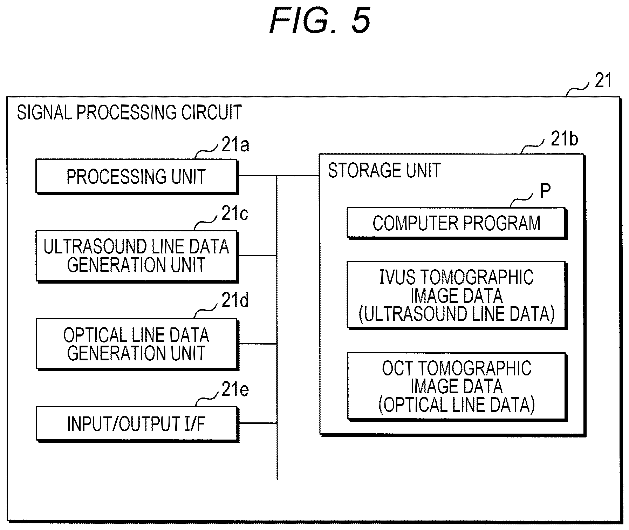

Fig. 5 is a block diagram illustrating a configuration example of the signal processing circuit 21. The signal processing circuit 21 is a computer, and includes a processing unit 21a, a storage unit 21b, an ultrasound line data generation unit 21c, an optical line data generation unit 21d, and an input/output I/F 21e. The processing unit 21a is constituted using one or a plurality of arithmetic processing units such as a central processing unit (CPU), a micro-processing unit (MPU), a graphics processing unit (GPU), a general-purpose computing on graphics processing unit (GPGPU), a field programmable gate array (FPGA) and SoC FPGA. The processing unit 21a is connected to each of the hardware components constituting the signal processing circuit 21 via a bus.

The storage unit 21b includes, for example, a main storage unit and an auxiliary storage unit. The main storage unit, which is a temporary storage area such as a static random access memory (SRAM), a dynamic random access memory (DRAM), or a flash memory, temporarily stores data necessary for the processing unit 21a to execute arithmetic processing. The auxiliary storage unit is a storage device such as a hard disk, an electrically erasable programmable ROM (EEPROM), or a flash memory. The storage unit 21b stores a computer program P (program product) to be executed by the processing unit 21a and various kinds of data necessary for other kinds of processing. Note that the auxiliary storage unit may be an external storage device connected to the signal processing circuit 21. The computer program P may be written in the auxiliary storage unit in a manufacturing stage of the signal processing circuit 21, or the computer program P distributed by a remote server apparatus may be acquired by the signal processing circuit 21 through communication and stored in the auxiliary storage unit. The computer program P may be readably recorded in a recording medium such as a magnetic disk, an optical disk, or a semiconductor memory, or may be read from the recording medium by a reading unit and stored in the auxiliary storage unit.

The ultrasound line data generation unit 21c samples the reflected wave signal of the ultrasound output from the ultrasound transmitter and receiver 12b of the image diagnosis catheter 1 at a predetermined rate to generate digital ultrasound line data from the reflected signal. The processing unit 21a stores the generated ultrasound line data in the storage unit 21b as IVUS tomographic image data.

The optical line data generation unit 21d samples the detection signal output from the light source device 3 at a predetermined rate to generate digital optical line data from the detection signal. The processing unit 21a stores the generated optical line data in the storage unit 21b as OCT tomographic image data.

The input/output I/F 21e is an interface to which the wireless communication circuit 25 is connected. The processing unit 21a controls operation of the wireless communication circuit 25 via the input/output I/F 21e and transmits and receives various kinds of data and information.

The processing unit 21a of the signal processing circuit 21 reads and executes the computer program P stored in the storage unit 21b, thereby executing processing of generating ultrasound line data in the ultrasound line data generation unit 21c and wirelessly transmitting the generated ultrasound line data to the external medical device 4 through the wireless communication circuit 25. In addition, the processing unit 21a can generate optical line data by the optical line data generation unit 21d by reading and executing the computer program P stored in the storage unit 21b and wirelessly transmit the generated optical line data to the external medical device 4 through the wireless communication circuit 25. By wirelessly transmitting the ultrasound line data and the optical line data to the external medical device 4, the IVUS tomographic image and the OCT image can be displayed on the external display apparatus 41.

In addition, the storage unit 21b stores the generated ultrasound line data and optical line data, and in a case where there is a request from the external medical device 4, the processing unit 21a can wirelessly transmit the requested ultrasound line data and optical line data to the external medical device 4.

Here, the IVUS image P1 and the OCT image P2 constructed from the optical line data and the ultrasound line data obtained by the optical transmitter and receiver 12a and the ultrasound transmitter and receiver 12b and the optical line data and the ultrasound line data will be described.

Fig. 6 is an explanatory diagram schematically illustrating a cross-section of the blood vessel through which the sensor unit 12 is inserted, and Figs. 7A and 7B are explanatory diagrams of the tomographic images. First, operation of the optical transmitter and receiver 12a and the ultrasound transmitter and receiver 12b in the blood vessel, and the optical line data and the ultrasound line data obtained by the optical transmitter and receiver 12a and the ultrasound transmitter and receiver 12b will be described with reference to Fig. 6.

When imaging of the tomographic image is started in a state where the sensor unit 12 and the shaft 13 are inserted into the blood vessel, the sensor unit 12 rotates about a central axis of the shaft 13 as a rotation center in a direction indicated by an arrow. In this event, the ultrasound transmitter and receiver 12b transmits and receives ultrasound at each rotation angle. Lines 1, 2,... 512 indicate transmission/reception directions of the ultrasound at each rotation angle. In the present embodiment, the ultrasound transmitter and receiver 12b intermittently transmits and receives ultrasound 512 times while rotating 360 degrees (one rotation) in the blood vessel. The ultrasound transmitter and receiver 12b acquires data of one line in the transmission/reception directions by transmitting and receiving ultrasound once, so that it is possible to obtain 512 pieces of ultrasound line data radially extending from the rotation center during one rotation. The 512 pieces of ultrasound line data are dense in the vicinity of the rotation center, but become sparse with distance from the rotation center. Thus, the image diagnosis apparatus 2 can construct a two-dimensional IVUS image P1 as illustrated in Fig. 7A by generating pixels in an empty space of each line by known interpolation processing.

Similarly, the optical transmitter and receiver 12a also transmits and receives near-infrared light (measurement light) at each rotation angle. The optical transmitter and receiver 12a also transmits and receives the measurement light 512 times while rotating 360 degrees in the blood vessel, so that it is possible to obtain 512 pieces of optical line data radially extending from the rotation center during one rotation. Moreover, for the optical line data, the image diagnosis apparatus 2 can construct a two-dimensional OCT image P2 illustrated in Fig. 7A by generating pixels in a vacant space of each line by known interpolation processing.

The two-dimensional tomographic image constructed from a plurality of pieces of ultrasound line data in this manner is referred to as the IVUS image P1 of one frame. In addition, a two-dimensional tomographic image constructed from a plurality of pieces of optical line data is referred to as the OCT image P2 of one frame. Note that, the sensor unit 12 scans while moving in the blood vessel, and thus, the IVUS image P1 or the OCT image P2 of one frame is acquired at each position rotated once within a movement range. In other words, the IVUS image P1 or the OCT image P2 of one frame is acquired at each position from the distal end side to the proximal end side of the probe 11 in the movement range, and thus, as illustrated in Fig. 7B, the IVUS image P1 or the OCT image P2 of a plurality of frames is acquired within the movement range.

Note that the number of times of transmission and reception of ultrasound and light in one rotation is an example, and the number of times of transmission and reception is not limited to 512 times. In addition, the number of times of transmission and reception of ultrasound and the number of times of transmission and reception of light may be the same or different.

As described above, according to the image diagnosis system 100 according to the present embodiment, the console can be eliminated, and the ultrasound line data and the optical line data can be generated by the small image diagnosis apparatus 2 and wirelessly transmitted to the external display apparatus.

In addition, the image diagnosis apparatus 2 and the light source device 3 are configured separately, and the light source device 3 is detachably attached to the image diagnosis apparatus 2, so that it is possible to downsize the image diagnosis apparatus 2. In a case where the user desires to capture an IVUS image, the user can remove the light source device 3 from the image diagnosis apparatus 2 and capture an IVUS image using the small image diagnosis apparatus 2. In a case where the user desires to capture an OCT image, the light source device 3 can be connected to the image diagnosis apparatus 2 to capture an OCT image. The light source device 3 is a device larger than the image diagnosis apparatus 2, but the user can dispose the light source device 3 under the bed. Thus, the light source device 3 does not interfere with procedure.

The connection cable 30 includes one cable, and the image diagnosis apparatus 2 and the light source device 3 can be connected by simply connecting one light source connector 37 to the light source device connection portion 29 of the image diagnosis apparatus 2. Specifically, the user can connect the image diagnosis apparatus 2 and the light source device 3 with the optical fiber 3a, the control signal line 3b, the detection signal line 3c, and the power supply line 3d by one connector connection.

The image diagnosis apparatus 2 includes an optical path length control device 24a that controls operation of the light source device 3, and the operation of the light source device 3 is controlled by the image diagnosis apparatus 2. The control and the signal processing circuit 21 are provided on the image diagnosis apparatus 2 side, and the optical system is provided in the light source device 3, so that it is possible to downsize the image diagnosis apparatus 2 and simplify the configuration of the light source device 3.

The image diagnosis apparatus 2 can operate with power supplied from the light source device 3. In addition, the image diagnosis apparatus 2 can operate with the power of the built-in battery 27.

The image diagnosis apparatus 2 incorporates the MDU 22, and can drive the probe 11 of the image diagnosis catheter 1 and perform image processing with one apparatus.

The image diagnosis apparatus 2 stores the generated ultrasound line data and optical line data in the storage unit 21b, and can provide the ultrasound line data and the optical line data stored in the storage unit 21b in response to a request from the external medical device 4.

Note that, in the present embodiment, the image diagnosis system 100 and the image diagnosis apparatus 2 that obtain a tomographic image of a blood vessel have been described. However, the image diagnosis system 100 and the image diagnosis apparatus 2 may be configured to capture a tomographic image of a luminal organ other than the blood vessel.

In addition, in the present embodiment, an example where a dual type catheter having functions of both intravascular ultrasound (IVUS) and optical coherence tomography (OCT) is used has been described. However, an IVUS catheter not including the optical transmitter and receiver 12a and including the ultrasound transmitter and receiver 12b, and an OCT catheter not including the ultrasound transmitter and receiver 12b and including the optical transmitter and receiver 12a can be connected to the image diagnosis apparatus 2 and used.

### Reference Signs List

- 1: Image diagnosis catheter
- 2: Image diagnosis apparatus
- 3: Light source device
- 3a: Optical fiber
- 3b: Control signal line
- 3c: Detection signal line
- 3d: Power supply line
- 4: External medical device
- 11: Probe
- 12: Sensor unit
- 13: Shaft
- 14: Guide wire insertion portion
- 15: Probe connector
- 20: Housing
- 21: Signal processing circuit
- 22b: Optical rotary joint (optical connection portion)
- 23: IVUS-related circuit
- 23a: Ultrasound signal transceiver
- 23b: Detector
- 23c: A/D converter
- 24: OCT-related circuit
- 24a: Optical path length control device
- 24b: A/D converter
- 25: Wireless communication circuit
- 26: Power supply circuit
- 27: Built-in battery
- 28: Probe connection portion
- 29: Light source device connection portion
- 29a: Optical fiber connection portion
- 29b: Control signal line connection portion
- 29c: Detection signal line connection portion
- 29d: Power supply connection portion
- 30: Connection cable
- 31: Wavelength sweeping light source
- 32: Optical path length variable mechanism
- 33: Optical coupler
- 34: Photoelectric conversion element
- 35: Demodulator
- 36: Power feeding circuit
- 37: Power supply connector
- 37a: Optical terminal
- 37b: Control signal terminal
- 37c: Detection signal terminal
- 37d: Power supply terminal
- 41: Display apparatus
- 100: Image diagnosis system
- P: Computer program

## Claims

1. A tomographic image generation apparatus that drives a sensor unit of a probe and generates a tomographic image of a luminal organ, the tomographic image generation apparatus comprising:
an ultrasound signal transceiver that transmits an ultrasound signal to the sensor unit and receives a reflected signal reflected by the luminal organ and output from the sensor unit;
a signal processing circuit that generates ultrasound tomographic image data of the luminal organ based on the reflected signal received by the ultrasound signal transceiver; and
a wireless communication circuit that transmits the ultrasound tomographic image data generated by the signal processing circuit to a display apparatus.

2. The tomographic image generation apparatus according to claim 1, further comprising:
a probe connection portion to which the probe is detachably connected;
a light source device connection portion to which an optical device is detachably connected, the optical device transmitting measurement light for obtaining an optical coherence tomographic image of the luminal organ and converting, into an electric signal, interference light based on reflected light reflected by the luminal organ and output from the sensor unit; and
an optical connection portion that optically connects the optical device and the probe, wherein
the signal processing circuit
generates the optical coherence tomographic image of the luminal organ based on the electric signal converted by the optical device, and
the wireless communication circuit
transmits optical coherence tomographic image data generated by the signal processing circuit to the display apparatus.

3. The tomographic image generation apparatus according to claim 2, further comprising:
an optical path length control device that outputs, to the optical device, a control signal for controlling an optical path length of reference light for obtaining the optical coherence tomographic image of the luminal organ.

4. The tomographic image generation apparatus according to claim 2, wherein the light source device connection portion includes:
a control signal line connection portion to which a control signal line for transmitting a control signal for controlling operation of the optical device is connected;
an optical fiber connection portion to which an optical fiber for transmitting the measurement light for obtaining the optical coherence tomographic image and the reflected light is connected; and
a detection signal line connection portion to which a detection signal line for transmitting the electric signal related to the interference light is connected.

5. The tomographic image generation apparatus according to claim 4, wherein
in the light source device connection portion
the control signal line connection portion, the optical fiber connection portion, and the detection signal line connection portion are connected to the control signal line, the optical fiber, and the detection signal line by one connector.

6. The tomographic image generation apparatus according to claim 4 or 5, wherein
the light source device connection portion includes
a power supply connection portion to which a power supply line through which power supplied from the optical device is transmitted is connected, and
the tomographic image generation apparatus includes
a battery mounted inside, and
a power supply circuit that, in a case where power supplied from an outside is received, supplies the received power to the signal processing circuit, and, in a case where the power supplied from the outside is not received, supplies power of the battery to the signal processing circuit.

7. The tomographic image generation apparatus according to claim 1 or 2, further comprising
a power supply circuit that supplies power of a battery mounted inside to the signal processing circuit.

8. The tomographic image generation apparatus according to claim 1 or 2, further comprising
a power supply circuit that receives power supplied from an outside and supplies the received power to the signal processing circuit.

9. The tomographic image generation apparatus according to claim 1 or 2, further comprising:
a rotation drive mechanism that rotates the sensor unit of the probe; and
a motor that applies power to the rotation drive mechanism.

10. The tomographic image generation apparatus according to claim 1, further comprising a storage unit that stores a plurality of pieces of ultrasound tomographic image data corresponding to a plurality of positions, in a longitudinal direction, of the luminal organ.

11. A tomographic image generation system comprising:
the tomographic image generation apparatus according to claim 2;
a catheter having a sensor unit that scans a luminal organ with ultrasound or light; and
an optical device that transmits measurement light for obtaining an optical coherence tomographic image of the luminal organ, receives interference light based on reflected light reflected by the luminal organ and output from the sensor unit, and converts the interference light into an electric signal.
